(19) Europäisches Patentamt
European Patent Office
Office européen des brevets

(11) Veröffentlichungsnummer: **0 056 082**
**A1**

(12) # EUROPÄISCHE PATENTANMELDUNG

(21) Anmeldenummer: **81108082.9**

(22) Anmeldetag: **08.10.81**

(51) Int. Cl.³: **C 07 C 102/08**, C 07 C 103/183

(30) Priorität: **18.12.80 DE 3047753**

(43) Veröffentlichungstag der Anmeldung: **21.07.82 Patentblatt 82/29**

(84) Benannte Vertragsstaaten: **BE CH DE FR GB LI NL**

(71) Anmelder: **Degussa Aktiengesellschaft, Weissfrauenstrasse 9, D-6000 Frankfurt am Main 1 (DE)**

(72) Erfinder: **Deller, Klaus, Dr., Dipl.-Chem., Friedhofstrasse 47, D-6452 Hainburg (DE)**
Erfinder: **Kleemann, Axel, Dr., Dipl.-Chem., Greifenhagenstrasse 25, D-6450 Hanau 9 (DE)**
Erfinder: **Martens, Jürgen, Dr., Dipl.-Chem., Hochstrasse 5, D-8755 Alzenau (DE)**
Erfinder: **Weigel, Horst, Odenwaldstrasse 56, D-6458 Rodenbach (DE)**

(54) **Verfahren zur Herstellung von 4-Amino-buttersäureamid-Hydrochlorid.**

(57) Verfahren zur Herstellung von 4-Amino-buttersäureamid-Hydrochlorid

4-Amino-buttersäureamid-Hydrochlorid wird in der Weise hergestellt, daß man 4-(Benzylamino)- oder 4-(Dibenzylamino)-butyronitril zu dem entsprechenden substituierten 4-Amino-buttersäureamid verseift und dieses in Form des Hydrochlorids in Gegenwart eines inerten Lösungsmittels und eines Platinmetall-Katalysators einer hydrierenden Behandlung unterwirft, wodurch die Benzylgruppe bzw. die beiden Benzylgruppen abgespalten werden.

EP 0 056 082 A1

0056082
80 267 AM

Degussa Aktiengesellschaft
Weissfrauenstrasse 9, 6000 Frankfurt 1

# Verfahren zur Herstellung von 4-Amino-buttersäureamid-Hydrochlorid

## Beschreibung

4-Amino-buttersäureamid und Derivate desselben haben grosse Bedeutung als Therapeutika, insbesondere als Neurotransmitter.

Gegenstand der Erfindung ist ein Verfahren zur Herstellung von 4-Amino-buttersäureamid-Hydrochlorid, welches dadurch gekennzeichnet ist, dass man

a) ein substituiertes Butyronitril der Formel

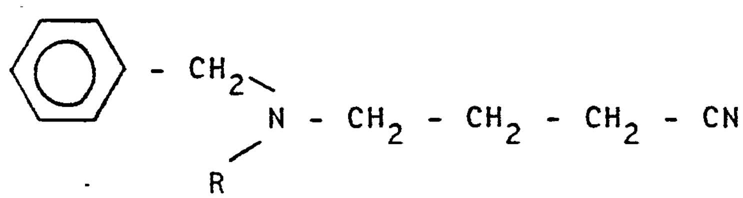

(I),

in der R Wasserstoff oder einen Benzylrest bedeutet, zu dem entsprechenden Buttersäureamid der Formel

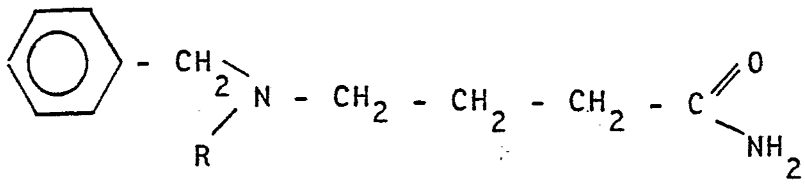 (II),

in der R wieder die bereits genannte Bedeutung hat, verseift und

b) das erhaltene Buttersäureamid der Formel (II) in Form des Hydrochlorids in Gegenwart eines inerten Lösungsmittels und eines Platinmetall-Katalysators einer hydrierenden Behandlung unterwirft.

Das als bevorzugtes Ausgangsmaterial dienende 4-(Benzylamino)-butyronitril und das 4-(Dibenzylamino)-butyronitril können in bekannter Weise durch Umsetzung von 4-Chlor-butyronitril mit Monobenzylamin bzw. Dibenzylamin hergestellt werden (vgl. J. Org. Chem., 32, 738 (1967); J. Chem. Soc. 1955, 2371). Sie können in Form der freien Base oder als Säureadditionssalze, z.B. als Hydrochloride, eingesetzt werden.

In der Reaktionsstufe a) werden die Butyronitrile der Formel (I) in an sich bekannter Weise zu den entsprechenden Buttersäureamiden der Formel (II) verseift, die bisher nicht beschrieben sind. Die Verseifung kann beispielsweise durch Einwirkung von 3 bis 30 gewichtsprozentigem Wasserstoffperoxid in alkalischem Medium

bei Temperaturen um 50°C oder durch Einwirkung von Braunstein in organischen Lösungsmitteln bei Raumtemperatur erfolgen.

Einfacher, schneller und vorteilhafter ist jedoch die saure Verseifung in Gegenwart von Mineralsäuren, wie Schwefelsäure oder Phosphorsäure und insbesondere Salzsäure oder Bromwasserstoffsäure, oder von Gemischen aus einer Halogenwasserstoffsäure, insbesondere Salzsäure oder Bromwasserstoffsäure, und Eisessig. Das Verseifungsmittel kann beispielsweise in einer Menge zwischen 200 und 1 000 ml, vorzugsweise zwischen 250 und 500 ml, pro Mol eingesetztem Butyronitril der Formel (I) angewandt werden. Zweckmässigerweise wird das Verseifungsmittel vorgelegt und dann bei einer Temperatur zwischen - 5 und + 10°C, vorzugsweise zwischen 0 und + 5°C, unter Rühren das Butyronitril der Formel (I) zugegeben. Zur Vervollständigung der Verseifungsreaktion wird noch eine Zeit lang, beispielsweise 2 Stunden lang, gerührt, wobei es sich empfiehlt, die Temperatur des Reaktionsgemisches unter 30°C zu halten.

Je nach den gewählten Verseifungsbedingungen fällt das gewünschte Buttersäureamid der Formel (II) in Form der freien Base oder als Säureadditionssalz, z.B. als Hydrochlorid oder Hydrobromid, an. Für die hydrierende Behandlung in der Reaktionsstufe b) wird es in Form des Hydrochlorids eingesetzt. Falls es also nicht bereits als Hydrochlorid vorliegt, wird es in an sich bekannter Weise durch Zusatz von Salzsäure oder durch Absorption an einem sauren Ionenaustauscher und Elution mit Salzsäure in das Hydrochlorid umgewandelt.

Die hydrierende Behandlung wird in Gegenwart eines inerten Lösungsmittels vorgenommen. Besonders geeignete

Lösungsmittel sind Wasser, primäre oder sekundäre Alkohole mit bis zu 6, insbesondere bis zu 4, Kohlenstoffatomen oder Gemische aus Wasser und solchen Alkoholen. Das Lösungsmittel kann beispielsweise in einer Menge zwischen 5 und 100 ml, vorzugsweise zwischen 10 und 100 ml, pro Gramm eingesetztem Buttersäureamid der Formel (II) angewandt werden.

Die hydrierende Behandlung erfordert ferner die Gegenwart eines Platinmetall-Katalysators. Besonders bevorzugte Katalysatoren sind Platin, Rhodium, Iridium oder Platin-IV-oxid und insbesondere Palladium. Es können auch Gemische der genannten Platinmetall-Katalysatoren angewandt werden. Die Katalysatoren können in freier Form oder als Trägerkatalysatoren (z.B. niedergeschlagen auf Aktivkohle) eingesetzt werden. Die einzusetzende Menge an Katalysatoren ist nicht kritisch. Zur Erzielung kurzer Reaktionszeiten empfiehlt es sich jedoch, den Platinmetall-Katalysator in einer Menge von 1 bis 100, vorzugsweise von 2 bis 10, Gewichtsprozent, bezogen auf eingesetztes Buttersäureamid der Formel (II), einzusetzen.

Die hydrierende Behandlung erfolgt zweckmässig bei einer Temperatur zwischen 0 und 100°C, vorzugsweise zwischen 40 und 60°C. Sie kann drucklos vorgenommen werden, indem man Wasserstoff durch das Reaktionsgemisch hindurchleitet, oder in einem druckfesten Reaktionsgefäss unter einem Wasserstoffdruck bis zu 100 bar. Vorzugsweise werden Wasserstoffdrucke bis zu 20 bar angewandt.

Bei der hydrierenden Behandlung werden die Benzylgruppe bzw. die Benzylgruppen aus dem Buttersäureamid der Fomel (II) in Form von Toluol abgespalten und es entsteht

unmittelbar in hoher Ausbeute das gewünschte 4-Amino-buttersäureamid-Hydrochlorid.

Durch die nachfolgenden Beispiele soll die Erfindung näher erläutert werden, und zwar wird in den Beispielen 1 bis 5 die Reaktionsstufe a) und in den Beispielen 6 bis 19 die Reaktionsstufe b) veranschaulicht. Soweit nicht anders angegeben, bedeuten die Prozentangaben Gewichtsprozente.

Beispiel 1:

Zu 25 ml konzentrierter Salzsäure gibt man bei 0 bis 5°C unter Rühren 21 g (0,1 Mol) 4-(Benzylamino)-butyronitril-Hydrochlorid. Man erwärmt das Reaktionsgemisch innerhalb einer Stunde unter Rühren auf 26°C. Bei dieser Temperatur lässt man noch eine Stunde nachreagieren. Die Lösung wird in 300 ml Isopropanol eingerührt. Dabei fällt ein farbloses Kristallisat aus, das man abnutscht. Das Kristallisat wird bei 50 bis 70°C im Wasserstrahlvacuum getrocknet. Die Ausbeute beträgt 16,9 g (73,9 % der Theorie) 4-(Benzylamino)-buttersäureamid-Hydrochlorid. Schmelzpunkt: 189 - 191°C Dünnschichtchromatogramm ($SiO_2$; Laufmittel n-Butanol : Eisessig : Wasser = 4 : 1 : 1) : $R_F$-Wert = 0,37

IR-Spektrum (KBr-Pressling): $\nu$ (Amid) 1645 und 1595 $cm^{-1}$. Im Bereich $\nu$ 2260 bis 2210 $cm^{-1}$, typisch für Nitril-Gruppen, ist kein Peak vorhanden.

Beispiel 2:

Zu 45 ml konzentrierter Salzsäure gibt man bei 0 bis 5°C unter Rühren 21 g (0,1 Mol) 4-(Benzylamino)-butyronitril-Hydrochlorid. Man erwärmt das Reaktionsgemisch innerhalb von zwei Stunden auf 28°C. Anschliessend neutralisiert man durch Zugabe von 50 %-iger Natronlauge bis pH 7. Das Lösungsmittel wird im Vacuum unter Erwärmen auf maximal 80°C verdampft. Das resultierende Gemisch aus Kochsalz und 4-(Benzylamino)-buttersäureamid-Hydrochlorid wird bei 60°C mit dreimal 150 ml absolutem Ethanol extrahiert und die vereinigten Ethanolextrakte werden eingeengt. Man erhält 18,5 g (80,9 %) 4-(Benzylamino)-buttersäureamid-Hydrochlorid als glänzende Schuppen. Schmelzpunkt: 189 - 191°C, nach Umkristallisieren aus Ethanol.

Beispiel 3:

Beispiel 1 wird wiederholt mit dem Unterschied, dass anstelle von Salzsäure ein Gemisch aus Eisessig und Bromwasserstoffsäure sowie anstelle des 4-(Benzylamino)-butyronitril-Hydrochlorids 0,1 Mol 4-(Benzylamino)-butyronitril als freie Base eingesetzt werden.
Die Ausbeute an 4-(Benzylamino)-buttersäureamid-Hydrobromid beträgt 20,3 g, entsprechend 74,3 % der Theorie.
Schmelzpunkt: 165 - 166°C.

| Elementaranalyse: | Berechnet (%) | | Gefunden (%) |
|---|---|---|---|
| $C_{11}H_{17}N_2OBr$ | C | 48,36 | 48,09 |
| (273,2) | H | 6,27 | 6,40 |
| | N | 10,26 | 10,21 |

## Beispiel 4:

Beispiel 2 wird wiederholt mit dem Unterschied, dass anstelle von Salzsäure 30 ml 35 %-iger Bromwasserstoffsäure sowie anstelle des 4-(Benzylamino)-butyronitril-Hydrochlorids 0,1 Mol 4-(Benzylamino)-butyronitril als freie Base eingesetzt werden.
Die Ausbeute an 4-(Benzylamino)-buttersäureamid-Hydrobromid beträgt 16,5 g, entsprechend 60,4 % der Theorie.
Schmelzpunkt: 165 - 167°C.

## Beispiel 5:

Beispiel 1 wird wiederholt mit dem Unterschied, dass anstelle von 4-(Benzylamino)-butyronitril-Hydrochlorid 30 g (0,1 Mol) 4-(Dibenzylamino)-butyronitril-Hydrochlorid eingesetzt werden.
Die Ausbeute an 4-(Dibenzylamino)-buttersäureamid-Hydrochlorid beträgt 15,0 g, entsprechend 47,2 % der Theorie.
IR-Spektrum (KBr-Pressling): $\nu$(Amid) 1650 und 1595 $cm^{-1}$

| Elementaranalyse: | | Berechnet (%) | Gefunden (%) |
|---|---|---|---|
| $C_{18}H_{23}ClN_2$ (318,9) | C | 67,85 | 67,52 |
| | H | 7,27 | 7,51 |
| | N | 8,79 | 8,49 |

Beispiel 6:

1 g 4-(Benzylamino)-buttersäureamid-Hydrochlorid wird in 90 ml Methanol gelöst, mit 0,1 g Palladium-Mohr versetzt und bei 40 bis 60°C unter Normaldruck mit Wasserstoffgas hydriert. Nach drei Stunden ist die Wasserstoffaufnahme beendet. Man filtriert den Katalysator ab und engt das Filtrat am Rotationsverdampfer zur Trockne ein. Der Rückstand wird aus Ethanol umkristallisiert. Ausbeute: 0,60 g (99 % der Theorie). Schmelzpunkt: 135 - 138°C.
$^1H$-NMR ($DMSO-d_6 + CDCl_3$): $\delta$ s 8,25 (3H, $NH_3$), s 7,55 (1 H, Amid), s 6,83 (1 H, Amid), m 2,83 (2 H, $CH_2$), m 2,22 (2 H, $CH_2$) und m 1,90 (2 H, $CH_2$) ppm.

Beispiel 7:

Beispiel 6 wird wiederholt mit dem einzigen Unterschied, dass anstelle des Methanols Ethanol als Lösungsmittel dient und der Katalysator bei einer Temperatur von 50 bis 60°C nach der Hydrierung abgenutscht wird.

Ausbeute: 0,58 g (95,7 % der Theorie)
Schmelzpunkt: 137 - 138°C

Beispiel 8:

Beispiel 7 wird wiederholt mit dem Unterschied, dass nur 10 ml Ethanol als Lösungsmittel dienen und bei

$10^{o}C$ filtriert wird. Der Filterkuchen (Gemisch aus 4-Amino-buttersäureamid-Hydrochlorid und Katalysator) wird mit 50 ml warmem Methanol behandelt und mit dem Filtrat vereinigt. Man engt zur Trockne ein und erhält nach dem Umkristallisieren aus Ethanol 0,59 g (97,4 % der Theorie) 4-Aminobuttersäureamid-Hydrochlorid. Schmelzpunkt: 134 - $137^{o}C$.

Beispiel 9:

Beispiel 6 wird wiederholt mit dem einzigen Unterschied, dass anstelle des Ethanols 50 ml Wasser als Lösungsmittel eingesetzt werden.
Ausbeute: 0,50 g (82,5 % der Theorie).
Schmelzpunkt: 133 - $136^{o}C$.

Beispiel 10:

Beispiel 6 wird wiederholt mit dem einzigen Unterschied, dass anstelle des Methanols 50 ml eines Gemisches aus gleichen Volumenteilen Wasser und Ethanol als Lösungsmittel eingesetzt werden.
Ausbeute: 0,52 g (85,8 % der Theorie).
Schmelzpunkt: 134 - $136^{o}C$.

Beispiel 11:

Beispiel 8 wird wiederholt mit dem einzigen Unterschied, dass anstelle des Ethanols 80 ml Isopropylalkohol als

Lösungsmittel eingesetzt werden.
Ausbeute: 0.49 g (80,9 % der Theorie).
Schmelzpunkt: 134 - 137°C.

Beispiel 12:

Beispiel 8 wird wiederholt mit dem einzigen Unterschied, dass anstelle des Ethanols 70 ml n-Butanol als Lösungsmittel eingesetzt werden.
Ausbeute: 0,49 g (80,9 % der Theorie)
Schmelzpunkt: 135 - 137°C.

Beispiel 13:

Beispiel 6 wird wiederholt mit dem einzigen Unterschied, dass anstelle von Palladium-Mohr 0,1 g metallisches Platin eingesetzt werden.
Ausbeute: 0,53 g (87,5 % der Theorie)
Schmelzpunkt: 136 - 138°C.

Beispiel 14:

Beispiel 8 wird wiederholt mit dem einzigen Unterschied, dass anstelle von Palladium-Mohr 0,05 g metallisches Rhodium eingesetzt werden.
Ausbeute: 0,53 g (87,5 % der Theorie)
Schmelzpunkt: 136 - 138°C.

Beispiel 15:

Beispiel 6 wird wiederholt mit dem einzigen Unterschied, dass anstelle von Palladium 0,2 g einer Präparation aus Palladium und Aktivkohle mit einem Edelmetallgehalt von 5 Gewichtsprozent eingesetzt werden.
Ausbeute: 0,49 g (80,9 % der Theorie)
Schmelzpunkt: 134 - 137°C.

Beispiel 16:

Beispiel 10 wird wiederholt mit dem einzigen Unterschied, dass anstelle von Palladium 0,1 g metallisches Iridium eingesetzt werden.
Ausbeute: 0,52 g (85,8 % der Theorie)
Schmelzpunkt: 135 - 138°C.

Beispiel 17:

Beispiel 10 wird wiederholt mit dem einzigen Unterschied, dass die Hydrierung in einem Schüttelautoklaven bei einem Wasserstoffdruck von 5 bar vorgenommen wird.
Ausbeute: 0,59 g (97,4 % der Theorie)
Schmelzpunkt: 135 - 137°C.

Beispiel 18:

Beispiel 6 wird wiederholt mit dem einzigen Unterschied, dass die Hydrierung in einem Schüttelautoklaven bei einem Wasserstoffdruck von 18 bar vorgenommen wird.
Ausbeute: 0,60 g (99,0 % der Theorie)
Schmelzpunkt: 136 - 139°C.

Beispiel 19:

Beispiel 6 wird wiederholt mit dem einzigen Unterschied, dass das Substrat 4-(Benzylamino)-buttersäureamid-Hydrochlorid ersetzt wird durch 1 g 4-(Dibenzylamino)-buttersäureamid-Hydrochlorid.
Ausbeute an 4-Aminobuttersäureamid-Hydrochlorid 0,41 g (96,8 % der Theorie).
Schmelzpunkt: 137 - 138°C.

0056082

80 267 AM

Degussa Aktiengesellschaft
Weissfrauenstrasse 9, 6000 Frankfurt 1

Verfahren zur Herstellung von 4-Amino-buttersäureamid-Hydrochlorid

## Patentansprüche:

1. Verfahren zur Herstellung von 4-Amino-buttersäureamid-Hydrochlorid, dadurch gekennzeichnet, dass man

a) ein substituiertes Butyronitril der Formel

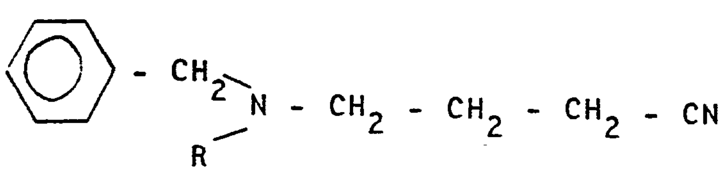

(I),

in der R Wasserstoff oder einen Benzylrest bedeutet, zu dem entsprechenden Buttersäureamid der Formel

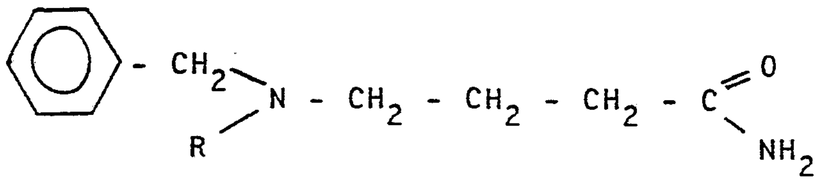 (II),

in der R wieder die bereits genannte Bedeutung hat, verseift und

b) das erhaltene Buttersäureamid der Formel (II) in Form des Hydrochlorids in Gegenwart eines inerten Lösungsmittels und eines Platinmetall-Katalysators einer hydrierenden Behandlung unterwirft.

2. Verfahren nach Anspruch 1, dadurch gekennzeichnet, dass man in der Reaktionsstufe a) die Verseifung mittels einer Mineralsäure oder eines Gemisches aus einer Halogenwasserstoffsäure und Eisessig bei einer Temperatur zwischen - 5 und + 30°C vornimmt.

3. Verfahren nach Anspruch 1 oder 2, dadurch gekennzeichnet, dass man in der Reaktionsstufe b) die hydrierende Behandlung in Gegenwart von Wasser, einem primären oder sekundären Alkohol mit bis zu 6 Kohlenstoffatomen, oder einem Gemisch aus Wasser und einem solchen Alkohol vornimmt.

4. Verfahren nach einem der Ansprüche 1 bis 3, dadurch gekennzeichnet, dass man in der Reaktionsstufe b) die hydrierende Behandlung in Gegenwart von Palladium vornimmt.

Europäisches Patentamt

# EUROPÄISCHER RECHERCHENBERICHT

0056082

Nummer der Anmeldung

EP 81 10 8082

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| Y | JOURNAL OF ORGANIC CHEMISTRY, Band 23, Heft 1, 27. Januar 1958, A. ZILKHA et al.: "Syntheses of DL-β-Aminobutyric Acid and Its N-Alkyl Derivatives", Seiten 94-96<br>* Seite 95 *<br>--- | 1 | C 07 C 102/08<br>C 07 C 103/183 |
| Y | FR - A - 1 109 586 (UCLAF)<br>* Zusammenfassung *<br>--- | 1 | |
| P/X | CHEMICAL ABSTRACTS, Band 95, Heft 19, 9. November 1981, Seite 676, Zusammenfassung 168496b, COLUMBUS, OHIO (US) K. DELLER et al.: "A new synthesis of 4-aminobutyramide" & Arch. Pharm. (Weinheim, Ger), 1981, 314(7), 648-9<br>* insgesamt *<br>------------ | 1-4 | RECHERCHIERTE SACHGEBIETE (Int. Cl.³)<br>C 07 C 102/00<br>C 07 C 103/00 |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 12.02.1982 | MOREAU |

EPA form 1503.1 06.78